# EUROPEAN PATENT APPLICATION

(11) **EP 4 516 332 A1**
(43) Date of publication of application: **05.03.2025**
(21) Application number: 23831506.3
(22) Date of filing: 28.06.2023
(51) Int. Cl.: A61M 1/16

(54) **BLOOD PURIFICATION DEVICE AND METHOD FOR CONTROLLING LIQUID FEED PUMP**

(30) Priority: 28.06.2022 JP 2022103782
(71) Applicant: Nikkiso Co., Ltd., Tokyo 150-6022 (JP)
(72) Inventor: KATAYAMA, Yuki, Makinohara-shi Shizuoka 421-0496 (JP)
(74) Representative: von Hirschhausen, Helge
(86) International application number: PCT/JP2023/023962
(87) International publication number: WO 2024/005066

(57) **Abstract**

The present invention comprises: a dialysate circuit 41 including a blood circuit 21, a dialysate feed channel 52, a dialysate discharge channel 53, and a bypass channel 54; a pressurization pump 73 installed on the dialysate circuit 41; and a main control unit 22 for controlling driving of the pressure pump 73. When a pressure difference is produced between the blood circuit 21 and the dialysate circuit 41 in instances where a switch is made from a bypass liquid feed mode in which liquid is fed from the dialysate feed channel 52 to the dialysate discharge channel 53 via the bypass channel 54 to a main liquid feed mode in which liquid is fed from the dialysate feed channel 52 to the dialysate discharge channel 53 via a dialyzer 10, the main control unit 22 adjusts the pump speed of the pressurization pump 73 so as to reduce the pressure difference.

## Description

### TECHNICAL FIELD

The present invention relates to a blood purification device and a method for controlling a liquid feed pump.

### BACKGROUND OF THE INVENTION

One of blood purification devices includes a bypass flow path (bypass line) that connects a supply-side line and a discharge-side line while bypassing a blood purifier (see Patent Literature 1). This blood purification device (dialysis machine) includes a blood purifier, a supply-side line through which dialysate to be supplied to the blood purifier flows, a discharge-side line through which the supplied dialysate flows, a pump means to make the dialysate flow from the supply-side line toward the discharge-side line, a bypass flow path that connects the supply-side line to the discharge-side line while bypassing the dialyzer, and a flow path switching means that switches the liquid feed mode between the first liquid feed mode of feeding the dialysate from the supply-side line to the discharge-side line via the dialyzer (hereinafter, referred to as the "main liquid feed mode") and the second liquid feed mode of feeding the dialysate from the supply-side line to the discharge-side line via the bypass flow path (hereinafter, referred to as the "bypass liquid feed mode"). This blood purification device switches to the bypass liquid feed mode to make the dialysate flow without passing through the dialyzer at any time other than during dialysis treatment, and switches to the main liquid feed mode during dialysis treatment to perform dialysis treatment. Such a configuration allows for avoidance of stagnation of the dialysate in the supply-side line and the discharge-side line during times other than dialysis treatment and also allows dialysis treatment to be resumed immediately.

### CITATION LIST

### PATENT LITERATURES

Patent Literature 1: JP 2010-29376A

### SUMMARY OF THE INVENTION

In conventional blood purification devices, when the liquid feed mode is switched from the bypass liquid feed mode to the main liquid feed mode, a sudden pressure fluctuation (so-called water hammer) occurs due to the pressure difference between the dialysate circuit and the blood circuit. This sudden pressure fluctuation may affect the flow control of the dialysate circuit, causing an error in the amount of water removed from the patient's blood.

Therefore, it is an object of the invention to provide a blood purification device and a drive control method for a liquid feed pump, which can reduce an adverse effect of a pressure fluctuation when the liquid feed mode is switched from the bypass liquid feed mode to the main liquid feed mode.

A blood purification device in an embodiment of the invention comprises:
a blood circuit to circulate blood of a patient through a blood purifier that purifies the blood;
a dialysate circuit which comprises a dialysate supply flow path to supply dialysate toward the blood purifier, a dialysate discharge flow path to discharge dialysate from the blood purifier, and a bypass flow path connecting the dialysate supply flow path to the dialysate discharge flow path, and through which liquid is fed in a liquid feed mode that is either a first liquid feed mode of feeding liquid from the dialysate supply flow path to the dialysate discharge flow path via the blood purifier, or a second liquid feed mode of feeding liquid from the dialysate supply flow path to the dialysate discharge flow path via the bypass flow path without passing through the blood purifier;
a liquid feed pump that is placed on the dialysate circuit and feeds liquid in the dialysate circuit;
a flow path switching unit that is placed on the dialysate circuit and switches the liquid feed mode between the first liquid feed mode and the second liquid feed mode; and
a drive control unit that controls the liquid feed pump and the flow path switching unit, wherein when a pressure difference occurs between the blood circuit and the dialysate circuit at the time that the flow path switching unit switches the liquid feed mode from the second liquid feed mode to the first liquid feed mode, the drive control unit adjusts drive of the liquid feed pump so that the pressure difference is reduced.

Furthermore, a blood purification device in an embodiment of the invention comprises:
a blood circuit to circulate blood of a patient through a blood purifier that purifies the blood;
a dialysate circuit which comprises a dialysate supply flow path to supply dialysate toward the blood purifier, a dialysate discharge flow path to discharge dialysate from the blood purifier, and a bypass flow path connecting the dialysate supply flow path to the dialysate discharge flow path, and through which liquid is fed in a liquid feed mode that is either a first liquid feed mode of feeding liquid from the dialysate supply flow path to the dialysate discharge flow path via the blood purifier, or a second liquid feed mode of feeding liquid from the dialysate supply flow path to the dialysate discharge flow path via the bypass flow path without passing through the blood purifier;
a liquid feed pump that is placed on the dialysate circuit and feeds liquid in the dialysate circuit;
a flow path switching unit that is placed on the dialysate circuit and switches the liquid feed mode between the first liquid feed mode and the second liquid feed mode; and
a drive control unit that controls the liquid feed pump and the flow path switching unit and performs a liquid feed mode switching operation to switch the liquid feed mode from the second liquid feed mode to the first liquid feed mode,
wherein the drive control unit comprises: a reception unit that receives a pressure of the blood circuit and a pressure of the dialysate circuit, an adjustment amount prediction unit that uses a prediction model trained by machine learning using pressure of the blood circuit, pressure of the dialysate circuit and an error in water removal amount in the liquid feed mode switching operation in the past as training data, and predicts, from the pressure of the blood circuit and the pressure of the dialysate circuit, a drive adjustment amount of the liquid feed pump that suppresses the error in water removal amount, and a drive adjustment unit that adjusts drive of the liquid feed pump according to the drive adjustment amount predicted by the adjustment amount prediction unit when the liquid feed mode is switched from the second liquid feed mode to the first liquid feed mode.

A method for controlling liquid feed pump in an embodiment of the invention comprises:
a drive control step of controlling drive of a liquid feed pump of a blood purification device that comprises a blood circuit to circulate blood of a patient through a blood purifier that purifies the blood, a dialysate circuit which comprises a dialysate supply flow path to supply dialysate toward the blood purifier, a dialysate discharge flow path to discharge dialysate from the blood purifier, and a bypass flow path connecting the dialysate supply flow path to the dialysate discharge flow path, and through which liquid is fed in a liquid feed mode that is either a first liquid feed mode of feeding liquid from the dialysate supply flow path to the dialysate discharge flow path via the blood purifier, or a second liquid feed mode of feeding liquid from the dialysate supply flow path to the dialysate discharge flow path via the bypass flow path without passing through the blood purifier, the liquid feed pump that is placed on the dialysate circuit and feeds liquid in the dialysate circuit, and a flow path switching unit that is placed on the dialysate circuit and switches the liquid feed mode between the first liquid feed mode and the second liquid feed mode,
wherein in the drive control step, when a pressure difference occurs between the blood circuit and the dialysate circuit at the time that the flow path switching unit switches the liquid feed mode from the second liquid feed mode to the first liquid feed mode, a command value of the liquid feed pump is adjusted so that the pressure difference is reduced.

Furthermore, a method for controlling liquid feed pump in an embodiment of the invention comprises:
a liquid feed mode switching step of performing a liquid feed mode switching operation to switch a liquid feed mode from a second liquid feed mode to a first liquid feed mode in a blood purification device that comprises a blood circuit to circulate blood of a patient through a blood purifier that purifies the blood, a dialysate circuit which comprises a dialysate supply flow path to supply dialysate toward the blood purifier, a dialysate discharge flow path to discharge dialysate from the blood purifier, and a bypass flow path connecting the dialysate supply flow path to the dialysate discharge flow path, and
through which liquid is fed in a liquid feed mode that is either the first liquid feed mode of feeding liquid from the dialysate supply flow path to the dialysate discharge flow path via the blood purifier, or the second liquid feed mode of feeding liquid from the dialysate supply flow path to the dialysate discharge flow path via the bypass flow path without passing through the blood purifier, a liquid feed pump that is placed on the dialysate circuit and feeds liquid in the dialysate circuit, and a flow path switching unit that is placed on the dialysate circuit and switches the liquid feed mode between the first liquid feed mode and the second liquid feed mode,
wherein the liquid feed mode switching step comprises performing a reception step of receiving a pressure of the blood circuit and a pressure of the dialysate circuit, an adjustment amount prediction step of using a prediction model trained by machine learning using pressure of the blood circuit, pressure of the dialysate circuit and an error in water removal amount in the liquid feed mode switching operation in the past as training data and predicting, from the pressure of the blood circuit and the pressure of the dialysate circuit, a drive adjustment amount of the liquid feed pump that suppresses the error in water removal amount, and a drive adjustment step of adjusting a command value of the liquid feed pump according to the drive adjustment amount predicted in the adjustment amount prediction step when the liquid feed mode is switched from the second liquid feed mode to the first liquid feed mode.

### Advantageous Effects of the Invention

According to an embodiment of the invention, it is possible to reduce an adverse effect of a pressure fluctuation when the liquid feed mode is switched from the bypass liquid feed mode to the main liquid feed mode.

### BRIEF DESCRIPTION OF DRAWINGS

Fig. 1A is a schematic configuration diagram illustrating a structure of a blood purification device in an embodiment of the present invention.
Fig. 2A is a graph showing pressures of a blood circuit and a dialysate circuit and error in water removal amount at the time of switching the liquid feed mode from the bypass liquid feed mode to the main liquid feed mode when a water removal error reduction operation is not performed.
Fig. 2B is a graph showing the pressures of the blood circuit and the dialysate circuit and pump speed of a pressure pump at the time of switching the liquid feed mode from the bypass liquid feed mode to the main liquid feed mode when the water removal error reduction operation is performed.
Fig. 2C is a graph showing the pressures of the blood circuit and the dialysate circuit and the error in water removal amount at the time of switching the liquid feed mode from the bypass liquid feed mode to the main liquid feed mode when the water removal error reduction operation is performed.
Fig. 3 is a flowchart showing the water removal error reduction operation.
Fig. 4 is a flowchart showing a modification of the water removal error reduction operation.
Fig. 5 is a schematic configuration diagram illustrating a structure of a blood purification device in the second embodiment.
Fig. 6A is a graph showing the pressures of the blood circuit and the dialysate circuit and the error in water removal amount at the time of switching the liquid feed mode from the bypass liquid feed mode to the main liquid feed mode when a water removal error reduction control is not performed.
Fig. 6B is a graph showing the pressures of the blood circuit and the dialysate circuit and the pump speed of the pressure pump at the time of switching the liquid feed mode from the bypass liquid feed mode to the main liquid feed mode in the second embodiment.
Fig. 6C is a graph showing the pressures of the blood circuit and the dialysate circuit and the error in water removal amount at the time of switching the liquid feed mode from the bypass liquid feed mode to the main liquid feed mode in the second embodiment.
Fig. 7 is an explanatory diagram illustrating a prediction model in the second embodiment.
Fig. 8 is a flowchart showing a liquid feed mode switching operation in the second embodiment.

### DETAILED DESCRIPTION OF THE INVENTION

A blood purification device in an embodiment of the invention will be described below with reference to the appended drawings. This blood purification device is a medical device that performs dialysis treatment to purify patient's blood using a dialyzer, and is a so-called hemodialysis machine. In particular, this blood purification device employs pump control that can reduce errors in water removal when switching from the bypass liquid feed mode to the main liquid feed mode.

### Configuration of Blood purification device

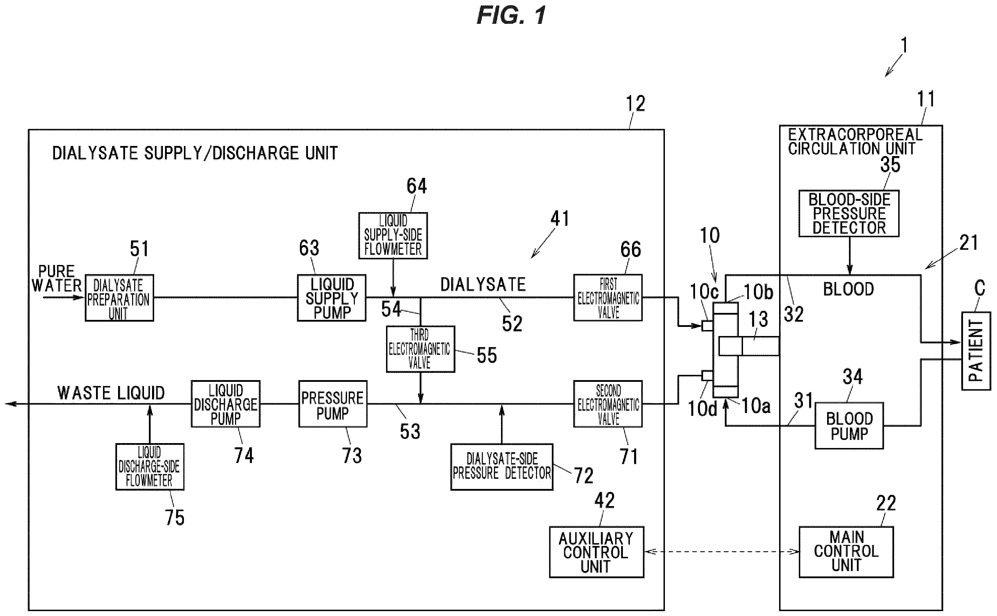

As shown in Fig. 1, a blood purification device 1 includes a dialyzer 10 that purifies blood of a patient C, an extracorporeal circulation unit 11 that circulates the blood of the patient C through the dialyzer 10, and a dialysate supply/discharge unit 12 that is connected to the dialyzer 10, supplies dialysate to the dialyzer 10 and discharges the dialysate from the dialyzer 10. The extracorporeal circulation unit 11 and the dialysate supply/discharge unit 12 are configured as separate units, and the dialyzer 10 is removably attached to the extracorporeal circulation unit 11 through a fixing tool 13.

The dialysis device 10 has a blood purification membrane (a hollow-fiber hemodialysis membrane, a hemodialysis filtration membrane, a flat hemodialysis membrane, or a hemofiltration membrane) thereinside. The blood purifier 10 also has a blood inlet 10a to introduce blood and a blood outlet 10b to discharge the introduced blood, as well as a dialysate inlet 10c to introduce dialysate and a dialysate outlet 10d to discharge the introduced dialysate. In the dialysis device 10, blood is purified by bringing the blood into contact with dialysate through the blood purification membrane. In addition, in the dialyzer 10, removal of water from the blood of the patient C is made possible by controlling the flow rate of liquid supplied to the dialyzer 10 and the flow rate of liquid discharged from the dialyzer 10. The dialyzer 10 is an example of the blood purifier.

The extracorporeal circulation unit 11 has a blood circuit 21 that circulates the blood of the patient C through the dialyzer 10, and a main control unit 22. The main control unit 22 will be described later.

The blood circuit 21 has an artery-side blood flow path 31 that is connected to the blood inlet 10a of the dialyzer 10 and leads the blood collected from a blood vessel of the patient C to the dialyzer 10, and a vein-side blood flow path 32 that is connected to the blood outlet 10b of the dialyzer 10 and returns the blood discharged from the dialyzer 10 to the blood vessel of the patient C. A blood pump 34 to circulate blood is placed on the artery-side blood flow path 31. A blood-side pressure detector 35 to detect pressure of the vein-side blood flow path 32 is placed on the vein-side blood flow path 32. The blood-side pressure detector 35 is an example of a pressure detection unit to detect pressure of the blood circuit 21, and detects the pressure of the blood circuit 21 by detecting the pressure of the vein-side blood flow path 32.

In the blood circuit 21, the blood from the patient C is led to the dialyzer 10 through the artery-side blood flow path 31 by driving the blood pump 34, and the blood is purified by the dialyzer 10 and is then returned to the patient C through the vein-side blood flow path 32. The blood of the patient C is thereby purified.

### Configuration of Dialysate supply/discharge unit

The dialysate supply/discharge unit 12 has a dialysate circuit 41 that supplies dialysate to the dialyzer 10 and also discharges the dialysate from the dialyzer 10, and an auxiliary control unit 42.

The dialysate circuit 41 has a dialysate preparation unit 51 that refines dialysate, a dialysate supply flow path 52 that is connected to the dialysate inlet 10c of the dialyzer 10 and supplies the dialysate refined by the dialysate preparation unit 51 toward the dialyzer 10, a dialysate discharge flow path 53 that is connected to the dialysate outlet 10d of the dialyzer 10 and collects and discharges the dialysate from the dialyzer 10, and a bypass flow path 54 that is connected to the dialysate supply flow path 52 and the dialysate discharge flow path 53 before and after the dialyzer 10 and connects the dialysate supply flow path 52 to the dialysate discharge flow path 53 while bypassing (avoiding) the dialyzer 10. That is, the dialysate circuit 41 is configured to feed dialysate in any one of the following liquid feed modes: the main liquid feed mode of feeding dialysate from the dialysate supply flow path 52 to the dialysate discharge flow path 53 via the dialyzer 10, and the bypass liquid feed mode of feeding dialysate from the dialysate supply flow path 52 to the dialysate discharge flow path 53 via the bypass flow path 54 without passing through the dialyzer 10. The main liquid feed mode is an example of the first liquid feed mode, and the bypass liquid feed mode is an example of the second liquid feed mode.

The dialysate preparation unit 51 prepares dialysate from pure water supplied thereto and a dialysate agent made of a concentrated solution or powder. The pure water supplied to the dialysate preparation unit 51 may be supplied from a pure water production unit mounted on the dialysate supply/discharge unit 12, or may be supplied from a pure water production unit provided outside the dialysate supply/discharge unit 12. In this regard, the dialysate preparation unit 51 can be omitted, and the configuration may be such that, e.g., dialysate is supplied to the dialysate supply/discharge unit 12 from an external dialysate supply device, etc.

From the upstream side, a liquid supply pump 63, a liquid supply-side flowmeter 64 and a first electromagnetic valve 66 are placed on the dialysate supply flow path 52. The liquid supply pump 63 is a liquid feed pump (a positive displacement diaphragm pump) that feeds the dialysate in the dialysate supply flow path 52. The dialysate is supplied to the dialyzer 10 by driving the liquid supply pump 63.

The liquid supply-side flowmeter 64 is a flowmeter that is placed on the downstream side of the liquid supply pump 63 and detects the flow rate in the dialysate supply flow path 52 (i.e., the flow rate of the liquid supplied to the dialyzer 10).

The first electromagnetic valve 66 is placed on the downstream side of a connection position connected to the bypass flow path 54, and is one of electromagnetic valves provided to perform switching of the liquid feed mode (flow path) between the main liquid feed mode and the bypass liquid feed mode.

From the upstream side, a second electromagnetic valve 71, a dialysate-side pressure detector 72, a pressure pump 73, a liquid discharge pump 74 and a liquid discharge-side flowmeter 75 are placed on the dialysate discharge flow path 53. The pressure pump 73 is an example of the liquid feed pump and the auxiliary pump.

The second electromagnetic valve 71 is placed on the upstream side of a connection position connected to the bypass flow path 54, and is one of the electromagnetic valves provided to perform switching of the liquid feed mode (flow path) between the main liquid feed mode and the bypass liquid feed mode.

The dialysate-side pressure detector 72 detects pressure of the dialysate discharge flow path 53. The dialysate-side pressure detector 72 is an example of a pressure detection unit to detect pressure of the dialysate circuit 41, and detects the pressure of the dialysate circuit 41 by detecting the pressure of the dialysate discharge flow path 53.

The liquid discharge pump 74 is a liquid feed pump (a positive displacement diaphragm pump) that feeds the dialysate in the dialysate discharge flow path 53. The pressure pump 73 is a cascade pump (a non-positive displacement pump) that feeds the dialysate in the dialysate discharge flow path 53 to assist liquid feeding by the liquid discharge pump 74. The dialysate from the dialyzer 10 is discharged by driving the liquid discharge pump 74 and the pressure pump 73. The flow rate of the supplied liquid to the dialyzer 10 and the flow rate of the discharged liquid from the dialyzer 10 are adjusted by controlling the liquid supply pump 63 and the liquid discharge pump 74, thereby controlling the amount of water removed from the blood of the patient C. With this blood purification device 1, dialysis treatment without water removal and dialysis treatment with water removal can be performed by controlling this water removal amount.

The liquid discharge-side flowmeter 75 is a flowmeter that is placed on the downstream side of the liquid discharge pump 74 and detects the flow rate in the dialysate discharge flow path 53 (i.e., the flow rate of the dialysate from the dialyzer 10).

The bypass flow path 54 is arranged such that one end is connected to the dialysate supply flow path 52 between the liquid supply-side flowmeter 64 and the first electromagnetic valve 66 and the other end is connected between the dialysate-side pressure detector 72 and the pressure pump 73. Thus, the bypass flow path 54 connects the dialysate supply flow path 52 to the dialysate discharge flow path 53 while bypassing the dialysate 10 without passing through the dialyzer 10. A third electromagnetic valve 55 is placed on the bypass flow path 54. The third electromagnetic valve 55 is an electromagnetic valve provided to perform, together with the first electromagnetic valve 66 and the second electromagnetic valve 71, switching of the liquid feed mode between the main liquid feed mode and the bypass liquid feed mode. That is, the first electromagnetic valve 66, the second electromagnetic valve 71 and the third electromagnetic valve 55 are examples of the flow path switching unit that switches the liquid feed mode between the main liquid feed mode and the bypass liquid feed mode. To switch the liquid feed mode from the main liquid feed mode to the bypass liquid feed mode, the first electromagnetic valve 66 and the second electromagnetic valve 71 are closed while opening the third electromagnetic valve 55. On the other hand, to switch the liquid feed mode from the bypass liquid feed mode to the main liquid feed mode, the first electromagnetic valve 66 and the second electromagnetic valve 71 are opened while closing the third electromagnetic valve 55.

The auxiliary control unit 42 communicates with the main control unit 22 of the extracorporeal circulation unit 11, and controls the liquid supply pump 63, the liquid discharge pump 74, the pressure pump 73 and each of the electromagnetic valves 55, 66 and 71 according to commands from the main control unit 22. The auxiliary control unit 42 is realized by appropriately combining an arithmetic element such as CPU, a memory, software, interface and a communication unit, etc.

### Description of Main control unit and its control

The main control unit 22 and the control by this main control unit 22 will now be described. The main control unit 22 is realized by appropriately combining an arithmetic element such as CPU, a memory, software, interface and a communication unit, etc., receives a detection value of the blood-side pressure detector 35 and controls drive of the blood pump 34. The main control unit 22 also communicates with the auxiliary control unit 42, receives detection values of the flowmeters 64, 75 and the dialysate-side pressure detector 72 through the auxiliary control unit 42, and controls drive of the liquid supply pump 63, the pressure pump 73 and the liquid discharge pump 74. Furthermore, the main control unit 22 communicates with the auxiliary control unit 42, and controls opening and closing of each of the electromagnetic valves 55, 66 and 71 through the auxiliary control unit 42. The main control unit 22 is an example of the drive control unit. In the present embodiment, the master-subordinate relationship between the control units 22 and 42 mounted on the extracorporeal circulation unit 11 and the dialysate supply/discharge unit 12 is such that the main control unit 22 mounted on the extracorporeal circulation unit 11 serves as the primary control unit and the auxiliary control unit 42 mounted on the dialysate supply/discharge unit 12 follows the commands of the main control unit 22. However, the master-subordinate relationship between the control units 22 and 42 may be such that the auxiliary control unit 42 mounted on the dialysate supply/discharge unit 12 serves as the primary control unit and the main control unit 22 mounted on the extracorporeal circulation unit 11 follows the commands of the auxiliary control unit 42, or there may be no master-subordinate relationship between the control units 22 and 42.

The main control unit 22 drives the blood pump 34, the liquid supply pump 63, the pressure pump 73 and the liquid discharge pump 74 in the state in which the liquid feed mode has been switched from the bypass liquid feed mode to the main liquid feed mode by opening and closing each of the electromagnetic valves 55, 66 and 71, and dialysis treatment is thereby performed. That is, during dialysis treatment, the blood pump 34 is driven to circulate blood through the dialyzer 10 and the liquid supply pump 63 is driven to supply dialysate to the dialyzer 10, while the pressure pump 73 and the liquid discharge pump 74 are driven to discharge the dialysate from dialyzer 10. During this dialysis treatment operation, the flow rate of liquid supplied to the dialyzer 10 (supply of dialysate) and the flow rate of liquid discharged from the dialyzer 10 (discharge of dialysate) are adjusted by controlling the liquid supply pump 63, the pressure pump 73 and the liquid discharge pump 74, thereby controlling the amount of water removed from the blood of the patient C. In the present embodiment, the pressure pump 73 is driven at a set pump speed (rotational speed), the liquid supply pump 63 is driven by feedback control based on the detection value of the liquid supply-side flowmeter 64 so as to achieve the target flow rate, and the liquid discharge pump 74 is driven by feedback control based on the detection value of the liquid discharge-side flowmeter 75 so as to achieve the target flow rate. The pump speed is an example of the command value of the liquid feed pump.

During preparation before dialysis treatment and during when dialysis treatment is temporarily stopped, the main control unit 22 makes the dialysate flow in the dialysate circuit 41 by driving the liquid supply pump 63, the pressure pump 73 and the liquid discharge pump 74 in the state in which the liquid feed mode has been switched from the main liquid feed mode to the bypass liquid feed mode by controlling opening and closing of each of the electromagnetic valves 55, 66 and 71. This operation will be called dialysate flow operation. During the dialysate flow operation, the pressure pump 73 is driven at a set pump speed, the liquid supply pump 63 is driven by feedback control based on the detection value of the liquid supply-side flowmeter 64 so as to achieve the target flow rate, and the liquid discharge pump 74 is driven by feedback control based on the detection value of the liquid discharge-side flowmeter 75 so as to achieve the target flow rate, in the same manner as during dialysis treatment.

### Description of Water removal error reduction operation

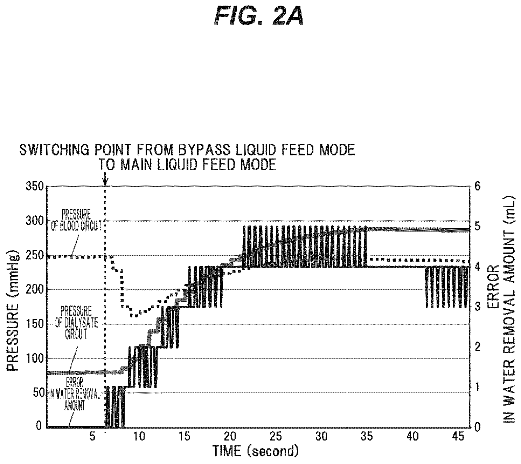

When transitioning from the dialysate flow operation to the dialysis treatment operation, the liquid feed mode is switched from the bypass liquid feed mode to the main liquid feed mode in the state in which each of the pumps 63, 73 and 74 on the dialysate circuit 41 is being driven, and at this time, a sudden pressure fluctuation occurs in the dialysate circuit 41 due to the difference between the pressure of the blood circuit 21 and the pressure of the dialysate circuit 41. This sudden pressure fluctuation affects the flow rate adjustment of the dialysate supply flow path 52 and the dialysate discharge flow path 53, causing an error in the amount of water removed from the blood of the patient C as shown in Fig. 2A. That is, when the pressure of the blood circuit 21 is greater than the pressure of the dialysate circuit 41, the pressure on the blood circuit 21 side propagates to the dialysate circuit 41 side at the time that the liquid feed mode is switched from the bypass liquid feed mode to the main liquid feed mode, resulting in that an error in water removal amount occurs due to excessive water removal, whereas when the pressure of the dialysate circuit 41 is greater than the pressure of the blood circuit 21, the pressure on the dialysate circuit 41 side propagates to the blood circuit 21 side at the time that the liquid feed mode is switched from the bypass liquid feed mode to the main liquid feed mode, resulting in that an error in water removal amount occurs due to fluid replacement. To address this, the main control unit 22 performs the water removal error reduction operation to reduce the error in the water removal amount due to the pressure fluctuation at the time of switching liquid feed mode from the bypass liquid feed mode to the main liquid feed mode when transitioning from the dialysate flow operation to the dialysis treatment operation. The water removal error reduction operation is an example of the drive control step and the method for controlling liquid feed pump.

In the water removal error reduction operation, the main control unit 22 adjusts the pump speed of the pressure pump 73 based on the pressure difference between the blood circuit 21 and the dialysate circuit 41 so that the pressure difference is reduced. In the present embodiment, TMP (Trans Membrane Pressure, i.e., trans-membrane pressure difference) is used as the pressure difference. TMP is the pressure difference between the pressure on the dialysate side and the pressure on the blood side in the dialyzer 10. In particular, the main control unit 22 detects the pressures of the blood circuit 21 and the dialysate circuit 41 by means of the blood-side pressure detector 35 and the dialysate-side pressure detector 72, and calculates the TMP in the dialyzer 10 based on the detected pressures of the blood circuit 21 and the dialysate circuit 41. Then, the pump speed of the pressure pump 73 is adjusted so that the calculated TMP is reduced. For example, a correction value is calculated by multiplying the calculated TMP by a predetermined coefficient, and the pump speed of the pressure pump 73 is increased or decreased by the correction value.

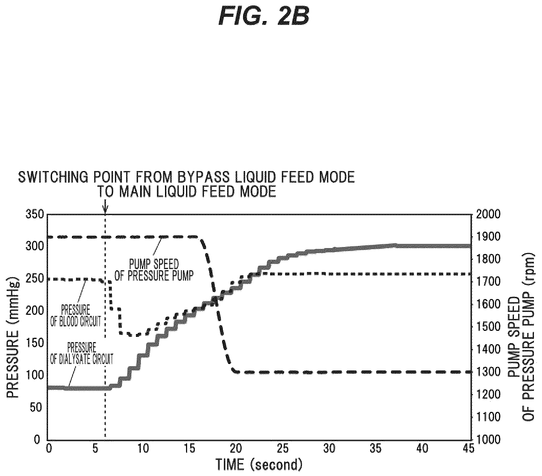

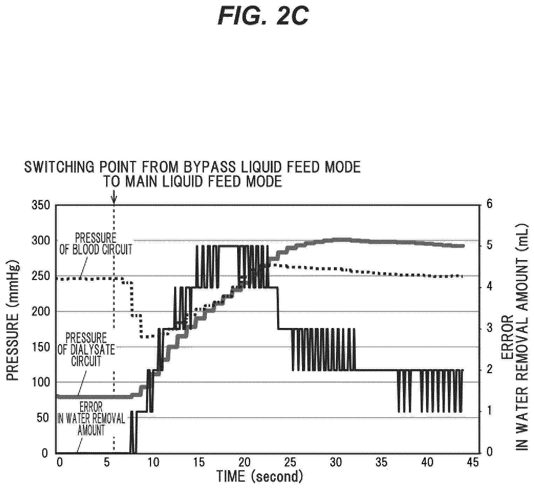

In the present embodiment, the pressure of the blood circuit 21 is basically higher than the pressure of the dialysate circuit 41 before switching to the main liquid feed mode and immediately after switching the flow path. Therefore, the pump speed of the pressure pump 73 is decreased from the original set value by the calculated correction value, as shown in Fig. 2B. This increases the pressure of the dialysate circuit 41 and reduces the TMP. Thus, it is possible to reduce the error in the amount of water removed from the blood of the patient C, as shown in Fig. 2C.

In addition, in the present embodiment, when a certain pressure difference occurs between the blood circuit 21 and the dialysate circuit 41 after switching the liquid feed mode from the bypass liquid feed mode to the main liquid feed mode, the above-described adjustment of the pump speed of the pressure pump 73 is performed. In particular, the pressure of the dialysate circuit 41, which is correlated with said pressure difference, is monitored by the dialysate-side pressure detector 72, and when the pressure becomes more than a predetermined threshold value (200 mmHg or 26.66 kPa in the present embodiment), the above-described adjustment of the pump speed of the pressure pump 73 is performed. In other words, an increase in the pressure of the dialysate circuit 41 triggers the adjustment of the pump speed of the pressure pump 73. Alternatively, the configuration may be such that instead of the increase in the pressure of the dialysate circuit 41, an increase in the TMP or an increase in the difference between the pressure of the blood circuit 21 and the pressure of the dialysate circuit 41 triggers the adjustment of the pump speed of the pressure pump 73.

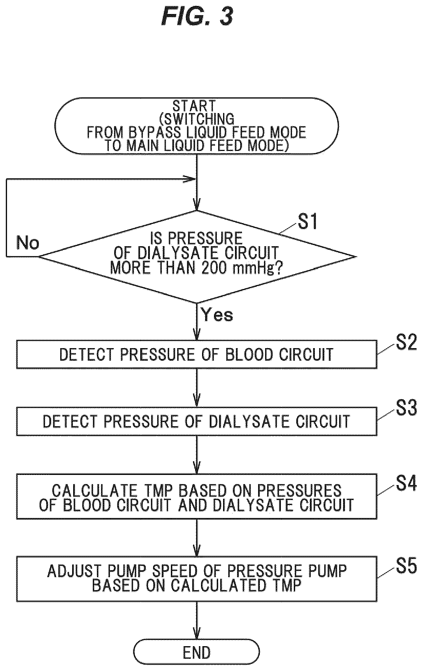

Here, an operational procedure of the water removal error reduction operation will be described with reference to Fig. 3. As shown in Fig. 3, when the liquid feed mode is switched from the bypass liquid feed mode to the main liquid feed mode, the water removal error reduction operation is performed due to this switching.

As shown in Fig. 3, after switching the flow path from the bypass flow path 54 to the main flow path, the main control unit 22 monitors the pressure of the dialysate circuit 41 using the dialysate-side pressure detector 72 (S1). Then, when the pressure of the dialysate circuit 41 becomes more than 200 mmHg (S1: Yes), the blood-side pressure detector 35 detects the pressure in the blood circuit 21 (S2) and the dialysate-side pressure detector 72 detects the pressure of the dialysate circuit 41 (S3). After detecting the pressures of the blood circuit 21 and the dialysate circuit 41, the TMP in the dialyzer 10 is calculated based on both the detected pressures (S4). After that, based on the calculated TMP, the pump speed of the pressure pump 73 is adjusted so that the TMP is reduced (S5). The water removal error reduction operation is then ended.

### Functions and Effects of the embodiment

In the configuration of the embodiment described above, when a pressure difference occurs between the blood circuit 21 and the dialysate circuit 41 at the time of switching the liquid feed mode from the bypass liquid feed mode to the main liquid feed mode, the pump speed of the pressure pump 73 is adjusted so that the pressure difference is reduced, thereby reducing the pressure difference between the blood circuit 21 and the dialysate circuit 41 at the time of switching the liquid feed mode from the bypass liquid feed mode to the main liquid feed mode. This makes it possible to reduce the water removal error (the error in water removal amount) due to the pressure fluctuation when switching the liquid feed mode from the bypass liquid feed mode to the main liquid feed mode.

In addition, since the pump speed of the pressure pump 73 among the plural pumps 63, 73 and 74 placed on the dialysate circuit 41 is adjusted in the water removal error reduction operation, it is possible to reduce the water removal error without changing the control of the liquid supply pump 63 and the liquid discharge pump 74 which are cooperatively driven to control the water removal amount which is the difference between the supplied liquid and the discharged liquid. This allows the control of water removal amount using the liquid supply pump 63 and the liquid discharge pump 74 and the control for the water removal error reduction using the pressure pump 73 to be easily performed in parallel, and it is also possible to prevent a trouble in coordination of the liquid supply pump 63 and the liquid discharge pump 74 caused by changing the control and its adverse effect on the control of water removal amount.

### Modifications

Although the embodiment of the invention has been described, the invention according to claims is not to be limited to the embodiment described above. Further, please note that not all combinations of the features described in the embodiment are necessary to solve the problem of the invention.

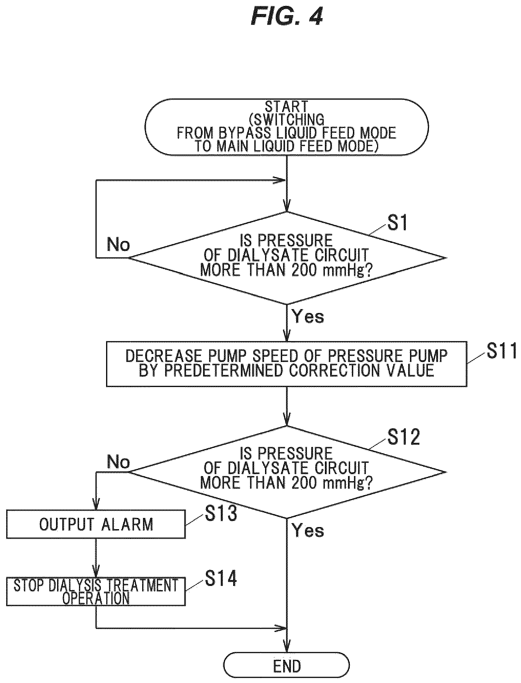

For example, in the embodiment described above, the configuration is such that in the water removal error reduction operation, the pressure difference (TMP) between the blood circuit 21 and the dialysate circuit 41 is acquired and the pump speed of the pressure pump 73 is adjusted using a correction value based on tis pressure difference. However, the configuration may be such that the pump speed of the pressure pump 73 is adjusted using a predetermined correction value as long as the pump speed of the pressure pump 73 is adjusted so that the pressure difference is reduced. For example, as shown in Fig. 4, the detection of pressure (S2, S3), the calculation of pressure difference (S4) and the adjustment of pump speed based on the pressure difference (S5) are omitted, and a step of decreasing the pump speed of the pressure pump 73 by a predetermined correction value (S11) is performed instead of these steps. In such a case, after the adjustment of the pump speed (S11), it is determined whether or not the pressure of the dialysate circuit 41 is more than 200 mmHg (S12), and when it is determined that the pressure of the dialysate circuit 41 is not more than 200 mmHg (S12: No), an alarm is output (S13) and the dialysis treatment operation is stopped (S14).

In the embodiment described above, the configuration is such that the pump speed of the pressure pump 73 is adjusted in the water removal error reduction operation, but it is not limited thereto as long as it is configured to adjust the command value of the liquid feed pump placed of the dialysate circuit 41. For example, it may be configured to adjust the command value of either the liquid supply pump 63 or the liquid drainage pump 74 instead of the pressure pump 73. Further, it may be configured to adjust the command values of plural liquid feed pumps instead of adjusting the command value of a single liquid feed pump.

In addition, in the embodiment described above, the configuration is such that the TMP in the dialyzer 10 is used as the pressure difference between the blood circuit 21 and the dialysate circuit 41 in the water removal error reduction operation, but it is not limited thereto. It may be configured to use, e.g., the difference between the pressure of the blood circuit 21 and the pressure of the dialysate circuit 41. In this case, the pressure of the blood circuit 21 may be detected by the blood-side pressure detector 35 and the pressure of the dialysate circuit 41 by the dialysate-side pressure detector 72.

In addition, in the embodiment described above, the configuration is such that detection of the pressures of the blood circuit 21 and the dialysate circuit 41 and adjustment of the command value of the pressure pump 73 based thereon are performed after switching the liquid feed mode from the bypass liquid feed mode to the main liquid feed mode. However, the configuration may be such that detection of the pressures of the blood circuit 21 and the dialysate circuit 41 and adjustment of the command value of the pressure pump 73 based thereon are performed before switching the liquid feed mode from the bypass liquid feed mode to the main liquid feed mode.

In addition, in the embodiment described above, the invention is applied to the blood purification device 1 that controls the flow rate of liquid supplied to the dialyzer 10 and the flow rate of liquid discharged from the dialyzer 10 using the separately provided liquid supply pump 63 and liquid discharge pump 74. However, the invention may be applied also to a blood purification device that controls the flow rate of liquid supplied to the dialyzer 10 and the flow rate of liquid discharged from the dialyzer 10 using a duplex pump that includes the liquid supply pump 63 and the liquid discharge pump 74 as one unit. In such a case, the configuration is such that a liquid feed pump is provided on the dialysate circuit 41 separately from the duplex pump, and when a pressure difference occurs between the blood circuit 21 and the dialysate circuit 41, the command value of this liquid feed pump is adjusted in the water removal error reduction operation so that the pressure difference is reduced.

In addition, in the embodiment described above, the configuration is such that the pressure of the dialysate circuit 41 is adjusted in the water removal error reduction operation by adjusting the drive of the pressure pump 73 placed on the dialysate circuit 41. However, the pressure of the blood circuit 21 may be adjusted instead. In this regard, however, since actively changing the pressure of the blood circuit 21 places a load on the blood (hemolysis, etc.), it is desirable to adjust the pressure of the dialysate circuit 41.

### Second embodiment

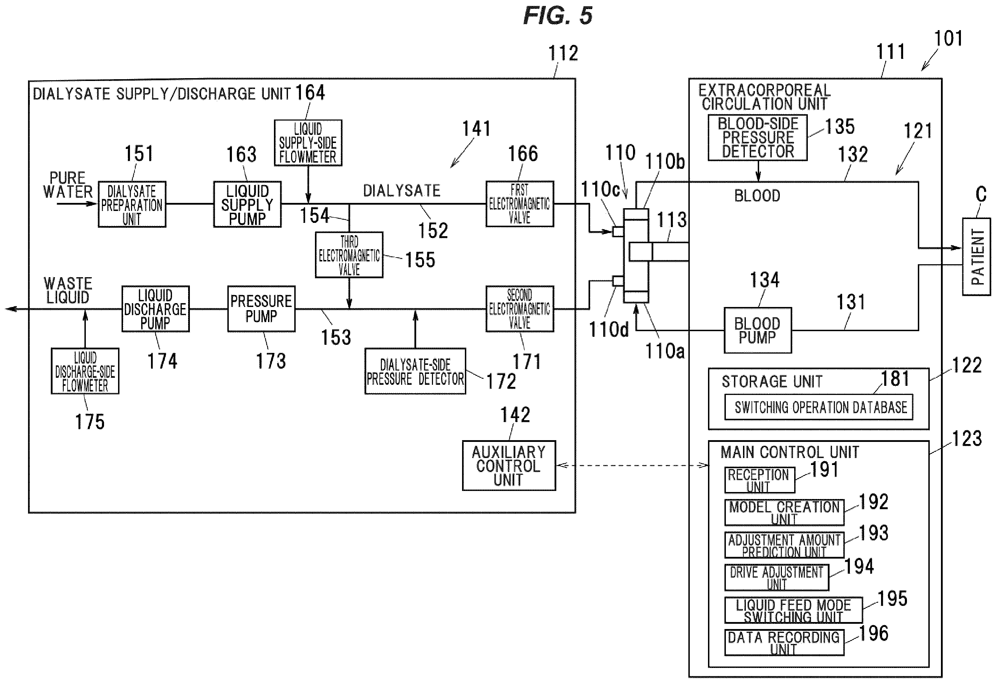

Next, a blood purification device 101 in the second embodiment of the invention will be described with reference to Figs. 5 to 8. This blood purification device 101 is a medical device that performs dialysis treatment to purify the blood of the patient C using a dialyzer 110, and is a so-called hemodialysis machine. In particular, this blood purification device 101 employs pump control that can reduce water removal errors when switching from the bypass liquid feed mode to the main liquid feed mode.

### Configuration of Blood purification device in the second embodiment

As shown in Fig. 5, the blood purification device 101 includes the dialyzer 10 that purifies the blood of the patient C, an extracorporeal circulation unit 111 that circulates the blood of the patient C through the dialyzer 110, and a dialysate supply/discharge unit 121 that is connected to the dialyzer 110, supplies dialysate to the dialyzer 110 and discharges the dialysate from the dialyzer 110. The extracorporeal circulation unit 111 and the dialysate supply/discharge unit 112 are configured as separate units, and the dialyzer 110 is removably attached to the extracorporeal circulation unit 111 through a fixing tool 113.

The dialyzer 110 has a blood purification membrane (a hollow-fiber hemodialysis membrane, a hemodialysis filtration membrane, a flat hemodialysis membrane, or a hemofiltration membrane) thereinside. The dialyzer 110 also has a blood inlet 110a to introduce blood and a blood outlet 110b to discharge the introduced blood, as well as a dialysate inlet 110c to introduce dialysate and a dialysate outlet 110d to discharge the introduced dialysate. In the dialyzer 110, blood is purified by bringing the blood into contact with dialysate through the blood purification membrane. In addition, in the dialyzer 110, removal of water from the blood of the patient C is made possible by controlling the flow rate of liquid supplied to the dialyzer 110 and the flow rate of liquid discharged from the dialyzer 110. The dialyzer 110 is an example of the blood purifier.

The extracorporeal circulation unit 111 has a blood circuit 121 that circulates the blood of the patient C through the dialyzer 110, a storage unit 122, and a main control unit 123. The storage unit 122 and the main control unit 123 will be described later.

The blood circuit 121 has an artery-side blood flow path 131 that is connected to the blood inlet 110a of the dialyzer 110 and leads the blood collected from a blood vessel of the patient C to the dialyzer 110, and a vein-side blood flow path 132 that is connected to the blood outlet 110b of the dialyzer 110 and returns the blood discharged from the dialyzer 110 to the blood vessel of the patient C. A blood pump 134 to circulate blood is placed on the artery-side blood flow path 131. A blood-side pressure detector 135 to detect pressure of the vein-side blood flow path 132 is placed on the vein-side blood flow path 132. The blood-side pressure detector 135 is an example of a pressure detection unit to detect pressure of the blood circuit 121, and detects the pressure of the blood circuit 121 by detecting the pressure of the vein-side blood flow path 132.

In the blood circuit 121, the blood from the patient C is led to the dialyzer 110 through the artery-side blood flow path 131 by driving the blood pump 134, and the blood is purified by the dialyzer 110 and is then returned to the patient C through the vein-side blood flow path 132. The blood of the patient C is thereby purified.

Configuration of Dialysate supply/discharge unit in the second embodiment The dialysate supply/discharge unit 112 has a dialysate circuit 141 that supplies dialysate to the dialyzer 110 and also discharges the dialysate from the dialyzer 110, and an auxiliary control unit 142.

The dialysate circuit 141 has a dialysate preparation unit 151 that refines dialysate, a dialysate supply flow path 152 that is connected to the dialysate inlet 110c of the dialyzer 110 and supplies the dialysate refined by the dialysate preparation unit 151 toward the dialyzer 110, a dialysate discharge flow path 153 that is connected to the dialysate outlet 110d of the dialyzer 110 and collects and discharges the dialysate from the dialyzer 110, and a bypass flow path 154 that is connected to the dialysate supply flow path 152 and the dialysate discharge flow path 153 before and after the dialyzer 110 and connects the dialysate supply flow path 152 to the dialysate discharge flow path 153 while bypassing (avoiding) the dialyzer 110. That is, the dialysate circuit 141 is configured to feed dialysate in any one of the following liquid feed modes: the main liquid feed mode of feeding dialysate from the dialysate supply flow path 152 to the dialysate discharge flow path 153 via the dialyzer 110, and the bypass liquid feed mode of feeding dialysate from the dialysate supply flow path 152 to the dialysate discharge flow path 153 via the bypass flow path 154 without passing through the dialyzer 110. The main liquid feed mode is an example of the first liquid feed mode, and the bypass liquid feed mode is an example of the second liquid feed mode.

The dialysate preparation unit 151 prepares dialysate from pure water supplied thereto and a dialysate agent made of a concentrated solution or powder. The pure water supplied to the dialysate preparation unit 151 may be supplied from a pure water production unit mounted on the dialysate supply/discharge unit 112, or may be supplied from a pure water production unit provided outside the dialysate supply/discharge unit 112. In this regard, the dialysate preparation unit 151 can be omitted, and the configuration may be such that, e.g., dialysate is supplied to the dialysate supply/discharge unit 112 from an external dialysate supply device, etc.

From the upstream side, a liquid supply pump 163, a liquid supply-side flowmeter 164 and a first electromagnetic valve 166 are placed on the dialysate supply flow path 152. The liquid supply pump 163 is a liquid feed pump (a positive displacement diaphragm pump) that feeds the dialysate in the dialysate supply flow path 152. The dialysate is supplied to the dialyzer 110 by driving the liquid supply pump 163.

The liquid supply-side flowmeter 164 is a flowmeter that is placed on the downstream side of the liquid supply pump 163 and detects the flow rate in the dialysate supply flow path 152 (i.e., the flow rate of the liquid supplied to the dialyzer 110).

The first electromagnetic valve 166 is placed on the downstream side of a connection position connected to the bypass flow path 154, and is one of electromagnetic valves provided to perform switching of the liquid feed mode (flow path) between the main liquid feed mode and the bypass liquid feed mode.

From the upstream side, a second electromagnetic valve 171, a dialysate-side pressure detector 172, a pressure pump 173, a liquid discharge pump 174 and a liquid discharge-side flowmeter 175 are placed on the dialysate discharge flow path 153. The pressure pump 173 is an example of the liquid feed pump and the auxiliary pump.

The second electromagnetic valve 171 is placed on the upstream side of a connection position connected to the bypass flow path 154, and is one of the electromagnetic valves provided to perform switching of the liquid feed mode (flow path) between the main liquid feed mode and the bypass liquid feed mode.

The dialysate-side pressure detector 172 detects pressure of the dialysate discharge flow path 153. The dialysate-side pressure detector 172 is an example of a pressure detection unit to detect pressure in the dialysate circuit 141, and detects the pressure of the dialysate circuit 141 by detecting the pressure of the dialysate discharge flow path 153.

The liquid discharge pump 174 is a liquid feed pump (a positive displacement diaphragm pump) that feeds the dialysate in the dialysate discharge flow path 153. The pressure pump 173 is a cascade pump (a non-positive displacement pump) that feeds the dialysate in the dialysate discharge flow path 153 to assist liquid feeding by the liquid discharge pump 174. The dialysate from the dialyzer 110 is discharged by driving the liquid discharge pump 174 and the pressure pump 173. The flow rate of the supplied liquid to the dialyzer 110 and the flow rate of the discharged liquid from the dialyzer 110 are adjusted by controlling the liquid supply pump 163 and the liquid discharge pump 174, thereby controlling the amount of water removed from the blood of the patient C. With this blood purification device 101, dialysis treatment without water removal and dialysis treatment with water removal can be performed by controlling this water removal amount.

The liquid discharge-side flowmeter 175 is a flowmeter that is placed on the downstream side of the liquid discharge pump 174 and detects the flow rate in the dialysate discharge flow path 153 (i.e., the flow rate of the dialysate from the dialyzer 110).

The bypass flow path 154 is arranged such that one end is connected to the dialysate supply flow path 152 between the liquid supply-side flowmeter 164 and the first electromagnetic valve 166 and the other end is connected between the dialysate-side pressure detector 172 and the pressure pump 173. Thus, the bypass flow path 154 connects the dialysate supply flow path 152 to the dialysate discharge flow path 153 while bypassing the dialysate 110 without passing through the dialyzer 110. A third electromagnetic valve 155 is placed on the bypass flow path 154. The third electromagnetic valve 155 is an electromagnetic valve provided to perform, together with the first electromagnetic valve 166 and the second electromagnetic valve 171, switching of the liquid feed mode between the main liquid feed mode and the bypass liquid feed mode. That is, the first electromagnetic valve 166, the second electromagnetic valve 171 and the third electromagnetic valve 155 are examples of the flow path switching unit that switches the liquid feed mode between the main liquid feed mode and the bypass liquid feed mode. To switch the liquid feed mode from the main liquid feed mode to the bypass liquid feed mode, the first electromagnetic valve 166 and the second electromagnetic valve 171 are closed while opening the third electromagnetic valve 155. On the other hand, to switch the liquid feed mode from the bypass liquid feed mode to the main liquid feed mode, the first electromagnetic valve 166 and the second electromagnetic valve 171 are opened while closing the third electromagnetic valve 155.

The auxiliary control unit 142 communicates with the main control unit 123 of the extracorporeal circulation unit 111, and controls the liquid supply pump 163, the liquid discharge pump 174, the pressure pump 173 and each of the electromagnetic valves 155, 166 and 171 according to commands from the main control unit 123. The auxiliary control unit 142 is realized by appropriately combining an arithmetic element such as CPU, a memory, software, interface and a communication unit, etc.

### Description of Storage unit and Main control unit

The storage unit 122 and the main control unit 123 of the extracorporeal circulation unit 111 will now be described. The storage unit 122 is composed of a memory such as a flash ROM, and stores various data. Particularly, the storage unit 122 stores switching operation database 181, as shown in Fig. 5.

The switching operation database 181 is database about a liquid feed mode switching operation to switch the liquid feed mode from the bypass liquid feed mode to the main liquid feed mode and stores the pressure of the blood circuit 121, the pressure of the dialysate circuit 141, pump speed adjustment coefficient and error in the water removal amount (hereinafter, referred to as the water removal error) in the liquid feed mode switching operations in the past. That is, the pressure of the blood circuit 121, the pressure of the dialysate circuit 141, the pump speed adjustment coefficient and the water removal error in each of the past liquid feed mode switching operations are stored. The pump speed adjustment coefficient is an example of the drive adjustment amount used to adjust the pump speed of the pressure pump 173 during the liquid feed mode switching operation, and is a numerical value expressing the drive adjustment amount as a multiplier applied to the original pump speed. As described in detail below, this blood purification device 101 has a configuration to reduce the water removal error by adjusting the pump speed of the pressure pump 173 at the time of switching the liquid feed mode from the bypass liquid feed mode to the main liquid feed mode. That is, the pump speed adjustment coefficient is a coefficient indicating an increase or decrease in adjustment of the pump speed to reduce the water removal errors. In addition, these pieces of information stored in the switching operation database 181 are also used to calculate (predict) the optimal pump speed adjustment coefficient in the liquid feed mode switching operation.

The main control unit 123 is realized by appropriately combining an arithmetic element such as CPU, a memory, software, interface and a communication unit, etc., receives a detection value of the blood-side pressure detector 135 and controls drive of the blood pump 134. The main control unit 123 also communicates with the auxiliary control unit 142, receives detection values of the flowmeters 164, 175 and the dialysate-side pressure detector 172 through the auxiliary control unit 142, and controls drive of the liquid supply pump 163, the pressure pump 173 and the liquid discharge pump 174. Furthermore, the main control unit 123 communicates with the auxiliary control unit 142, and controls opening and closing of each of the electromagnetic valves 155, 166 and 171 through the auxiliary control unit 142. The main control unit 123 is an example of the drive control unit. In the second embodiment, the master-subordinate relationship between the control units 123 and 142 mounted on the extracorporeal circulation unit 111 and the dialysate supply/discharge unit 112 is such that the main control unit 123 mounted on the extracorporeal circulation unit 111 serves as the primary control unit and the auxiliary control unit 142 mounted on the dialysate supply/discharge unit 112 follows the commands of the main control unit 123. However, the master-subordinate relationship between the control units 123 and 142 may be such that the auxiliary control unit 142 mounted on the dialysate supply/discharge unit 112 serves as the primary control unit and the main control unit 123 mounted on the extracorporeal circulation unit 111 follows the commands of the auxiliary control unit 142, or there may be no master-subordinate relationship between the control units 123 and 142.

After performing the above-described liquid feed mode switching operation to switch the liquid feed mode from the bypass liquid feed mode to the main liquid feed mode, the main control unit 123 drives the blood pump 134, the liquid supply pump 163, the pressure pump 173 and the liquid discharge pump 174 in this state, and dialysis treatment is thereby performed. That is, during dialysis treatment, the blood pump 134 is driven to circulate blood through the dialyzer 110 and the liquid supply pump 163 is driven to supply dialysate to the dialyzer 110, while the pressure pump 173 and the liquid discharge pump 174 are driven to discharge the dialysate from dialyzer 110. During this dialysis treatment operation, the flow rate of liquid supplied to the dialyzer 110 (supply of dialysate) and the flow rate of liquid discharged from the dialyzer 110 (discharge of dialysate) are adjusted by controlling the liquid supply pump 163, the pressure pump 173 and the liquid discharge pump 174, thereby controlling the amount of water removed from the blood of the patient C. In the second embodiment, the pressure pump 173 is driven at a set pump speed (rotational speed), the liquid supply pump 163 is driven by feedback control based on the detection value of the liquid supply-side flowmeter 164 so as to achieve the target flow rate, and the liquid discharge pump 174 is driven by feedback control based on the detection value of the liquid discharge-side flowmeter 175 so as to achieve the target flow rate. The pump speed is an example of the command value of the liquid feed pump.

During preparation before dialysis treatment and during when dialysis treatment is temporarily stopped, the main control unit 123 makes the dialysate flow in the dialysate circuit 141 by driving the liquid supply pump 163, the pressure pump 173 and the liquid discharge pump 174 in the state in which the liquid feed mode has been switched from the main liquid feed mode to the bypass liquid feed mode by controlling opening and closing of each of the electromagnetic valves 155, 166 and 171. This operation will be called dialysate flow operation. During the dialysate flow operation, the pressure pump 173 is driven at a set pump speed, the liquid supply pump 163 is driven by feedback control based on the detection value of the liquid supply-side flowmeter 164 so as to achieve the target flow rate, and the liquid discharge pump 174 is driven by feedback control based on the detection value of the liquid discharge-side flowmeter 175 so as to achieve the target flow rate, in the same manner as during dialysis treatment.

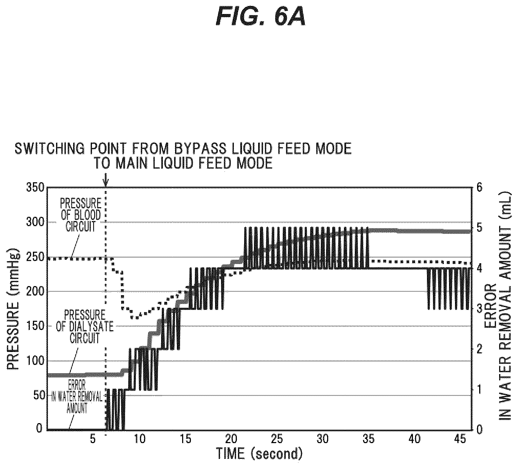

When transitioning from the dialysate flow operation to the dialysis treatment operation, the liquid feed mode is switched from the bypass liquid feed mode to the main liquid feed mode in the state in which each of the pumps 163, 173 and 174 on the dialysate circuit 141 is being driven, and at this time, a sudden pressure fluctuation occurs in the dialysate circuit 141 due to the difference between the pressure of the blood circuit 121 and the pressure of the dialysate circuit 141. This sudden pressure fluctuation affects the flow rate adjustment of the dialysate supply flow path 152 and the dialysate discharge flow path 153, causing an error in the amount of water removed from the blood of the patient C as shown in Fig. 6A. That is, when the pressure of the blood circuit 121 is greater than the pressure of the dialysate circuit 141, the pressure on the blood circuit 121 side propagates to the dialysate circuit 141 side at the time that the liquid feed mode is switched from the bypass liquid feed mode to the main liquid feed mode, resulting in that a water removal error occurs due to excessive water removal, whereas when the pressure of the dialysate circuit 141 is greater than the pressure of the blood circuit 121, the pressure on the dialysate circuit 141 side propagates to the blood circuit 121 side at the time that the liquid feed mode is switched from the bypass liquid feed mode to the main liquid feed mode, resulting in that a water removal error occurs due to fluid replacement. To address this, in the second embodiment, the main control unit 123 performs control to reduce the water removal error caused by the pressure fluctuation at the time of switching the liquid feed mode from the bypass liquid feed mode to the main liquid feed mode in the liquid feed mode switching operation. In this regard, as the configuration related to this liquid feed mode switching operation, the main control unit 123 functions as a reception unit 191, a model creation unit 192, an adjustment amount prediction unit 193, a drive adjustment unit 194, a liquid feed mode switching unit 195 and a data recording unit 196, as shown in Fig. 5.

The reception unit 191 receives the pressure of the blood circuit 121 by receiving the detection value of the blood-side pressure detector 135 and also receives the pressure of the dialysate circuit 141 by receiving the detection value of the dialysate-side pressure detector 172. In the second embodiment, the pressure of the blood circuit 121 and the pressure of the dialysate circuit 141 are received before switching the liquid feed mode from the bypass liquid feed mode to the main liquid feed mode in the liquid feed mode switching operation. For example, the pressures of the blood circuit 121 and the dialysate circuit 141 when an instruction to start dialysis treatment is given are received.

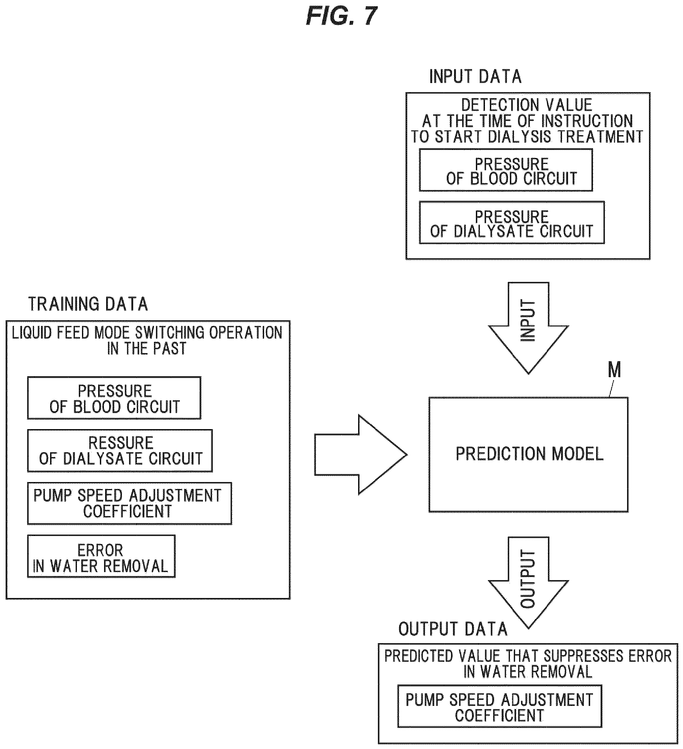

The model creation unit 192 performs machine learning on the pressure of the blood circuit 121, the pressure of the dialysate circuit 141, the pump speed adjustment coefficient and the water removal error of the past liquid feed mode switching operations stored in the switching operation database 181 as training data, and creates a prediction model M which takes the pressure of the blood circuit 121 and the pressure of the dialysate circuit 141 as input and from which a pump speed adjustment coefficient that suppresses the water removal errors is output (see Fig. 7). The water removal error at the time of the liquid feed mode switching operation is caused by the flow rate fluctuation due to the pressure difference between the pressure of the blood circuit 121 and the pressure of the dialysate circuit 141 as described above, hence, there is a strong correlation between the pressure of the blood circuit 121 and the pressure of the dialysate circuit 141 during the liquid feed mode switching operation and the water removal error during the liquid feed mode switching operation. In addition, variation in the water removal error during the liquid feed mode switching operation is caused by adjusting the pump speed of the pressure pump 173 at the time of switching from the main liquid feed mode to the bypass liquid feed mode. Therefore, for example, the prediction model M predicts, based on data (the pressure of blood circuit 121, the pressure of dialysate circuit 141, the pump speed adjustment coefficient and the water removal error) of the past liquid feed mode switching operations extracted from the switching operation database 181, a relational expression between the pump speed adjustment coefficient and the water removal error for the input pressures of the blood circuit 121 and the dialysate circuit 141, and based on this relational expression, calculates and outputs the pump speed adjustment coefficient that suppresses the water removal error. When, e.g., the pressure of the blood circuit 121 is greater than the pressure of the dialysate circuit 141, excessive water removal occurs, hence, the prediction model M predicts a numerical value to decrease the pump speed (less than 1) as the pump speed adjustment coefficient that suppresses the water removal error, and when the pressure of the dialysate circuit 141 is greater than the pressure of the blood circuit 121, fluid replacement occurs, hence, the prediction model M predicts a numerical value to increase the pump speed (more than 1) as the pump speed adjustment coefficient that suppresses the water removal error. Preferably, the prediction model M predicts a pump speed adjustment coefficient resulting in the water removal error of not more than 50 ml/h (preferably 0). In addition, in the second embodiment, the configuration is such that the pressures of the blood circuit 121 and the dialysate circuit 141 when an instruction to start dialysis treatment is given are used as the pressure of the blood circuit 121 and the pressure of the dialysate circuit 141 to be input data and training data. However, it may be configured to use the pressures of the blood circuit 121 and the dialysate circuit 141 obtained a few seconds after the instruction to start dialysis treatment is given. In addition, in the second embodiment, the configuration is such that the detection value of the blood-side pressure detector 135 and the detection value of the dialysate-side pressure detector 172 are used as the pressure of the blood circuit 121 and the pressure of the dialysate circuit 141 which are to be input data and training data. However, it may be configured to use detection values or predicted values of other detection units. In addition, regarding the data of the past liquid feed mode switching operations, it is preferable that only data from a certain period of time in the past (e.g., one year) be used as training data for machine learning. Machine learning is a technology in which a computer learns a large amount of data and automatically constructs algorithms or models to perform tasks such as classification and prediction, and, e.g., neural network can be used as a technique or algorithm to make machine learning work.

In addition, in the second embodiment, the prediction model M is created, assuming that the pre-adjustment pump speed of the pressure pump 173 at the time of the liquid feed mode switching operation is constant. That is, the prediction model M is created by taking into account a numerical value of this pre-adjustment constant pump speed.

The adjustment amount prediction unit 193 uses the prediction model M created by the model creation unit 192 and predicts, from the pressure of the blood circuit 121 and the pressure of the dialysate circuit 141, a pump speed adjustment coefficient that suppresses the water removal error. That is, the adjustment amount prediction unit 193 inputs the pressure of the blood circuit 121 and the pressure of the dialysate circuit 141, which are received by the reception unit 191, into the prediction model M and acquires the pump speed adjustment coefficient output from the prediction model M.

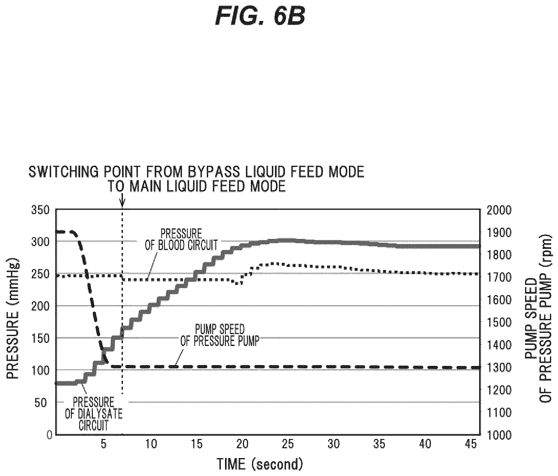

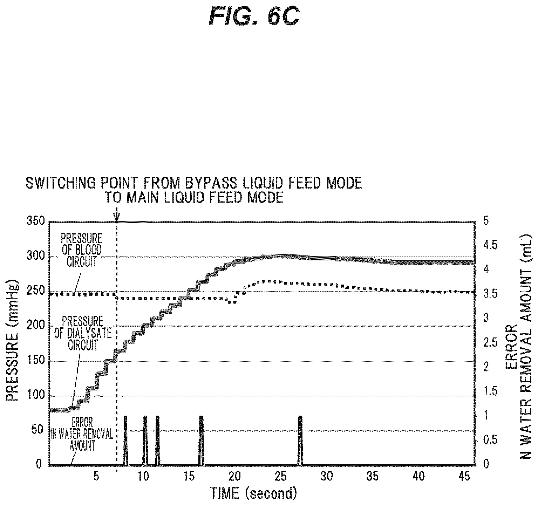

When there is a certain pressure difference between the blood circuit 121 and the dialysate circuit 141 at the time of switching the liquid feed mode from the bypass liquid feed mode to the main liquid feed mode, the drive adjustment unit 194 adjusts the drive of the pressure pump 173 using the pump speed adjustment coefficient predicted by the adjustment amount prediction unit 193. That is, the pump speed of the pressure pump 173 is multiplied by the predicted pump speed adjustment coefficient and the set value of the pump speed of the pressure pump 173 is changed to the multiplied value, thereby increasing or decreasing the pump speed of the pressure pump 173. When, e.g., the pressure of the blood circuit 121 is greater than the pressure of the dialysate circuit 141 as shown in Fig. 6B, excessive water removal occurs, hence, a numerical value to decrease the pump speed (less than 1) is predicted as the pump speed adjustment coefficient suppressing the water removal error by the prediction model M, and the set value of the pump speed of the pressure pump 173 is reduced (the pressure of the dialysate circuit 141 is increased) based on this predicted pump speed adjustment coefficient. The error in water removal from the blood of the patient C can thereby be reduced, as shown in Fig. 6C. Then, when, e.g. the pressure of the dialysate circuit 141 is greater than the pressure of the blood circuit 121, fluid replacement occurs, hence, a numerical value to increase the pump speed (more than 1) is predicted as the pump speed adjustment coefficient suppressing the water removal error by the prediction model M, and the set value of the pump speed of the pressure pump 173 is increased (the pressure of the dialysate circuit 141 is reduced) based on this predicted pump speed adjustment coefficient.

The liquid feed mode switching unit 195 switches the liquid feed mode from the bypass liquid feed mode to the main liquid feed mode by controlling opening and closing of each of the electromagnetic valves 155, 166 and 171. That is, the liquid feed mode switching unit 195 switches the liquid feed mode from the bypass liquid feed mode to the main liquid feed mode by controlling each of the electromagnetic valves 155, 166 and 171 so that the first electromagnetic valve 166 and the second electromagnetic valve 171 are opened and the third electromagnetic valve 155 is closed.

The data recording unit 196 records data (the pressure of the blood circuit 121, the pressure of the dialysate circuit 141, the pump speed adjustment coefficient and the water removal error) of the liquid feed mode switching operation into the switching operation database 181. That is, the flow rate of the liquid supplied to the dialyzer 110 and the flow rate of the liquid discharged from the dialyzer 110 are acquired by receiving the detection value of each of the flowmeters 164 and 175, and the water removal error is calculated based thereon. Then, the pressure of the blood circuit 121 and the pressure of the dialysate circuit 141 which are received by the reception unit 191, the pump speed adjustment coefficient predicted by the adjustment amount prediction unit 193, the calculated water removal error, and the date of implementation of the liquid feed mode switching operation are associated and recorded in the switching operation database 181. The water removal error is calculated based on the cumulative flow rate until a predetermined number of seconds (e.g., 45 seconds) has elapsed after switching the liquid feed mode to the main liquid feed mode, and a value of the water removal error accumulated until the predetermined number of seconds has elapsed is recorded.

### Description of Liquid feed mode switching operation

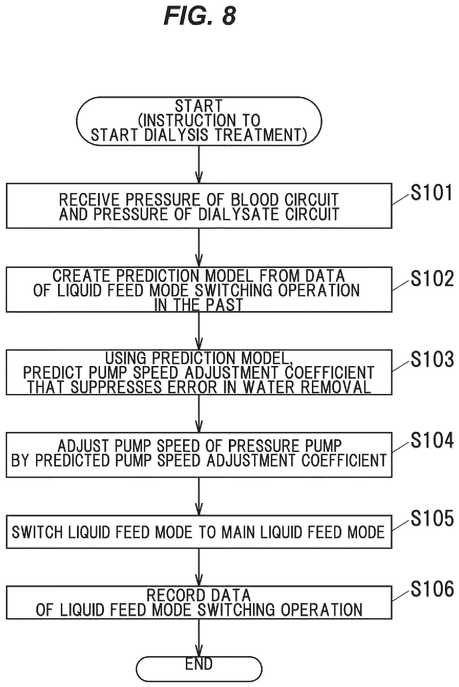

Here, an operational procedure of the liquid feed mode switching operation will be described with reference to Fig. 8. This liquid feed mode switching operation is performed in response to an instruction to start dialysis treatment, and is started in the state in which the dialysate flow operation is being performed. The liquid feed mode switching operation is an example of the drive control step and the method for controlling liquid feed pump.

In the liquid feed mode switching operation, first, the pressure of the blood circuit 121 and the pressure of the dialysate circuit 141 are received (S101) (the reception step), as shown in Fig. 8. That is, the pressure of the blood circuit 121 is received by receiving the detection value of the blood-side pressure detector 135 and the pressure of the dialysate circuit 141 is received by receiving the detection value of the dialysate-side pressure detector 172.

After receiving the pressures of the blood circuit 121 and the dialysate circuit 141, the prediction model M is created by the model creation unit 192 (S102) (the model creation step). That is, data (the pressure of blood circuit 121, the pressure of dialysate circuit 141, the pump speed adjustment coefficient and the water removal error) of the past liquid feed mode switching operations is extracted from the switching operation database 181, and the prediction model M is created by machine learning using the extracted data of the past liquid feed mode switching operations as training data.

After creating the prediction model M, the adjustment amount prediction unit 193 uses the prediction model M and a pump speed adjustment coefficient that suppresses the water removal error is predicted from the pressure of the blood circuit 121 and the dialysate circuit 141 (S103) (the adjustment amount prediction step). That is, the pressures of the blood circuit 121 and the dialysate circuit 141 received in S101 are input into the prediction model M created by the model creation unit 192, and the pump speed adjustment coefficient output from the prediction model M in response thereto is acquired as a predicted value.

After predicting the pump speed adjustment coefficient that suppresses the water removal error, the drive of the pressure pump 173 is adjusted by the drive adjustment unit 194 using the predicted pump speed adjustment coefficient (S104) (the drive adjustment step). That is, the pump speed of the pressure pump 173 is multiplied by the predicted pump speed adjustment coefficient, and the set value of the pump speed of the pressure pump 173 is changed to the multiplied value, thereby increasing or decreasing the pump speed of the pressure pump 173. After that, each of the electromagnetic valves 155, 166 and 171 is controlled by the liquid feed mode switching unit 195, thereby switching the liquid feed mode from the bypass liquid feed mode to the main liquid feed mode (S105). In this regard, the adjustment amount prediction step (S103) and the drive adjustment step (S104) are performed only when there is a certain pressure difference between the blood circuit 121 and the dialysate circuit 141. That is, when there is no certain pressure difference between the blood circuit 121 and the dialysate circuit 141, it is preferable to omit the adjustment amount prediction step (S103) and the drive adjustment step (S104).

After switching the liquid feed to the main liquid feed mode, the data of the liquid feed mode switching operation is recorded in the switching operation database 181 after waiting until a certain number of seconds (e.g., 45 seconds) has elapsed (S106). That is, the water removal error is calculated from the detection value of each of the flowmeters 164 and 175, and then, the pressure of the blood circuit 121 and the pressure of the dialysate circuit 141 received in S101, the pump speed adjustment coefficient predicted in S103, the calculated water removal error, and the date of implementation of the liquid feed mode switching operation are associated and recorded in the switching operation database 181. In case that S103 is omitted, the pump speed adjustment coefficient is 1. This liquid feed mode switching operation is thereby ended. In the second embodiment, the configuration is such that the prediction model M is created after starting the liquid feed mode switching operation. However, the configuration may be such that the prediction model M is created in advance before starting the liquid feed mode switching operation. For example, the configuration may be such that, at the end of the liquid feed mode switching operation, a prediction model M to be used for the next liquid feed mode switching operation is created. Furthermore, the configuration may be such that a common prediction model M is used in plural liquid feed mode switching operations.

### Functions and Effects of the embodiment

In the second embodiment described above, by configuring such that the pump speed adjustment coefficient that suppresses the water removal error is predicted based on the prediction model M created by training on data of the past liquid feed mode switching operations and the drive of the pressure pump 173 is adjusted using said pump speed adjustment coefficient at the time of switching the liquid feed mode from the bypass liquid feed mode to the main liquid feed mode, it is possible to reduce the water removal error due to the pressure fluctuation when switching the liquid feed mode from the bypass liquid feed mode to the main liquid feed mode. In addition, since it is configured such that the prediction model M is used and the pump speed adjustment coefficient that suppresses water removal errors is predicted from the pressures of the blood circuit 121 and the dialysate circuit 141, it is possible to use an optimal pump speed adjustment coefficient that takes into account the conditions of tube arrangement or the conditions of the patient C.

In addition, since machine learning is preformed so that the prediction model M is trained on only data of the past liquid feed mode switching operations over a certain period of time in the past (e.g., the last one year), it is possible to predict the pump speed adjustment coefficient taking into account deterioration of tubes, pumps, sensors, etc. over time. It is thereby possible to accurately predict the pump speed adjustment coefficient that suppresses the water removal error.

In addition, since it is configured such that the pump speed of the pressure pump 173, among the plural pumps 163, 173 and 174 placed on the dialysate circuit 141, is adjusted in the liquid feed mode switching operation, it is possible to reduce the water removal error without changing the control of the liquid supply pump 163 and the liquid discharge pump 174 which are cooperatively driven to control the water removal amount which is the difference between the supplied liquid and the discharged liquid. This allows the control of water removal amount using the liquid supply pump 163 and the liquid discharge pump 174 and the control for the water removal error reduction using the pressure pump 173 to be easily performed in parallel, and it is also possible to prevent a trouble in coordination of the liquid supply pump 163 and the liquid discharge pump 174 caused by changing the control and its adverse effect on the control of water removal amount.

### Modification of the second embodiment

Although the embodiment of the invention has been described, the invention according to claims is not to be limited to the embodiment described above. Further, please note that not all combinations of the features described in the embodiment are necessary to solve the problem of the invention.

For example, in the second embodiment described above, the prediction model M outputs the pump speed adjustment coefficient and the adjustment amount prediction unit 193 predicts the pump speed adjustment coefficient using the prediction model M, but the configuration is not limited thereto. For example, it may be configured to output and predict a post-adjustment pump speed obtained by multiplying the original pump speed by the pump speed adjustment coefficient, or it may be configured to output and predict an adjustment value to be subtracted from or added to the original pump speed.

In addition, in the second embodiment described above, the configuration is such that the pressure of the blood circuit 121 and the pressure of the dialysate circuit 141 are used as the input data and the training data. However, the configuration may be such that a numerical value indicating the pressure difference between the pressure of the blood circuit 121 and the pressure of the dialysate circuit 141 (e.g., a subtraction value or a division value between the pressure of the blood circuit 121 and the pressure of the dialysate circuit 141) is used instead as the input data and the training data.

In addition, in the second embodiment described above, the configuration is such that the data of the feed mode switching operations performed by the blood purification device 101 itself is used as the training data for the prediction model M. However, the configuration may be such that sample data for complement in case that number of implementations of the liquid feed mode switching operations by the blood purification device 101 itself is small is stored in the switching operation database 181.

In addition, in the second embodiment described above, the configuration is such that the data of the feed mode switching operations performed by the blood purification device 101 itself is used as the training data for the prediction model M. However, the configuration may be such that data of the feed mode switching operations performed by another blood purification device 101 is used as the training data for the prediction model M. In this regard, however, when considering prediction of the pump speed adjustment coefficient taking into account inherent characteristics of the device, it is desirable that only data of the liquid feed mode switching operations performed by the blood purification device 101 itself be used as the training data for the prediction model M.

In addition, in the second embodiment described above, the configuration is such that adjustment of the pump speed of the pressure pump 173 based on the pump speed adjustment coefficient is performed before switching the liquid feed mode to the main liquid feed mode in the liquid feed mode switching operation. However, the configuration may be such that the adjustment of the pump speed of the pressure pump 173 based on the pump speed adjustment coefficient is performed after switching the liquid feed mode to the main liquid feed mode in the liquid feed mode switching operation.

In addition, in the second embodiment described above, the configuration is such that the pump speed of the pressure pump 173 is adjusted in the liquid feed mode switching operation. However, it is not limited thereto as long as it is configured to adjust the command value of the liquid feed pump placed of the dialysate circuit 141. For example, it may be configured to adjust the command value of either the liquid supply pump 163 or the liquid drainage pump 74 instead of the pressure pump 173. Further, it may be configured to adjust the command values of plural liquid feed pumps instead of adjusting the command value of a single liquid feed pump.

In addition, in the second embodiment described above, the configuration is such that the pressure of the dialysate circuit 141 is adjusted by adjusting the drive of the liquid feed pump placed on the dialysate circuit 141 to reduce the water removal error. However, the configuration may be such that the pressure of the blood circuit 121 is adjusted instead by adjusting the drive of the liquid feed pump (e.g., the blood pump 134) placed on the blood circuit 121. In this regard, however, since actively changing the pressure of the blood circuit 121 places a load on the blood (hemolysis, etc.), it is desirable to adjust the pressure of the dialysate circuit 141.

In addition, in the second embodiment described above, the invention is applied to the blood purification device 101 that controls the flow rate of liquid supplied to the dialyzer 110 and the flow rate of liquid discharged from the dialyzer 110 using the separately provided liquid supply pump 163 and liquid discharge pump 174. However, the invention may be applied also to a blood purification device that controls the flow rate of liquid supplied to the dialyzer 110 and the flow rate of liquid discharged from the dialyzer 110 using a duplex pump that includes the liquid supply pump 163 and the liquid discharge pump 174 as one unit.

### Summary of the embodiments

Technical ideas understood from the embodiments will be described below citing the reference signs, etc., used for the embodiments. However, each reference sign, etc., described below is not intended to limit the constituent elements in the claims to the members, etc., specifically described in the embodiments.
(1) A blood purification device 1 comprising: a blood circuit 21 to circulate blood of a patient C through a blood purifier 10 that purifies the blood; a dialysate circuit 41 which comprises a dialysate supply flow path 52 to supply dialysate toward the blood purifier 10, a dialysate discharge flow path 53 to discharge dialysate from the blood purifier, and a bypass flow path 54 connecting the dialysate supply flow path 52 to the dialysate discharge flow path 53, and through which liquid is fed in a liquid feed mode that is either a first liquid feed mode of feeding liquid from the dialysate supply flow path 52 to the dialysate discharge flow path 53 via the blood purifier 10, or a second liquid feed mode of feeding liquid from the dialysate supply flow path 52 to the dialysate discharge flow path 53 via the bypass flow path 54 without passing through the blood purifier 10; a liquid feed pump 73 that is placed on the dialysate circuit 41 and feeds liquid in the dialysate circuit 41; a flow path switching unit 55, 66, 76 that is placed on the dialysate circuit 41 and switches the liquid feed mode between the first liquid feed mode and the second liquid feed mode; and a drive control unit 22 that controls the liquid feed pump 73 and the flow path switching unit 55, 66, 76, wherein when a pressure difference occurs between the blood circuit 21 and the dialysate circuit 41 at the time that the flow path switching unit 55, 66, 76 switches the liquid feed mode from the second liquid feed mode to the first liquid feed mode, the drive control unit 22 adjusts drive of the liquid feed pump 73 so that the pressure difference is reduced.
   It is thereby possible to reduce the water removal error due to the pressure fluctuation when switching the liquid feed mode from the bypass liquid feed mode to the main liquid feed mode.
(2) The blood purification device 1 as defined by (1), comprising: a liquid supply pump 63 that is placed on the dialysate supply flow path 52 and feeds the dialysate; a liquid discharge pump 74 that is placed on the dialysate discharge flow path 53 and feeds the dialysate; and an auxiliary pump 73 as the liquid feed pump that is placed on the dialysate discharge flow path 53 on an upstream side of the liquid discharge pump 74 to assist liquid feeding by the liquid discharge pump 74, wherein when the pressure difference occurs between the blood circuit 21 and the dialysate circuit 41 at the time that the flow path switching unit 55, 66, 76 switches the liquid feed mode from the second liquid feed mode to the first liquid feed mode, the drive control unit 22 adjusts drive of the auxiliary pump 73 so that the pressure difference is reduced.
   It is thereby possible to reduce the water removal error without changing the control of the liquid supply pump and the liquid discharge pump which are cooperatively driven. As a result, a trouble in coordination of the liquid supply pump and the liquid discharge pump caused by changing the control can be prevented.
(3) The blood purification device 1 as defined by (1) or (2), wherein the drive control unit 22 acquires the pressure difference, and adjusts drive of the liquid feed pump 73 based on the pressure difference at the time that the flow path switching unit 55, 66, 76 switches the liquid feed mode from the second liquid feed mode to the first liquid feed mode.
(4) The blood purification device 1 as defined by any one of (1) or (3), wherein the drive control unit 22 acquires a trans-membrane pressure difference in the blood purifier 10 as the pressure difference, and adjusts drive of the liquid feed pump 73 based on the trans-membrane pressure difference at the time that the flow path switching unit 55, 66, 76 switches the liquid feed mode from the second liquid feed mode to the first liquid feed mode.
   It is thereby possible to reduce the water removal error with higher accuracy.
(5) The blood purification device 1 as defined by any one of (1) to (4), wherein when a pressure of the dialysate circuit 41 becomes more than a predetermined threshold value at the time that the flow path switching unit 55, 66, 76 switches the liquid feed mode from the second liquid feed mode to the first liquid feed mode, the drive control unit 22 adjusts drive of the liquid feed pump 73 so that the pressure difference is reduced. Since adjustment of the drive of the liquid feed pump is triggered by the pressure of the dialysate circuit correlated with the pressure difference instead of the pressure difference itself, it is possible to omit acquisition of the pressure of the blood circuit and it is possible to perform the water removal error reduction operation more easily.
(6) The blood purification device 1 defined as any one of (1) to (5), wherein before the flow path switching unit 55, 66, 76 switches the liquid feed mode from the second liquid feed mode to the first liquid feed mode, the drive control unit 22 adjusts drive of the liquid feed pump 73 so that the pressure difference is reduced. It is thereby possible to reduce the pressure fluctuation itself when the liquid feed mode is switched from the bypass liquid feed mode to the main liquid feed mode.
(7) A blood purification device 101 comprising: a blood circuit 121 to circulate blood of a patient C through a blood purifier 110 that purifies the blood; a dialysate circuit 141 which comprises a dialysate supply flow path 152 to supply dialysate toward the blood purifier 110, a dialysate discharge flow path 153 to discharge dialysate from the blood purifier 110, and a bypass flow path 154 connecting the dialysate supply flow path 152 to the dialysate discharge flow path 152, and through which liquid is fed in a liquid feed mode that is either a first liquid feed mode of feeding liquid from the dialysate supply flow path 152 to the dialysate discharge flow path 153 via the blood purifier 110, or a second liquid feed mode of feeding liquid from the dialysate supply flow path 152 to the dialysate discharge flow path 153 via the bypass flow path 154 without passing through the blood purifier 110, a liquid feed pump 173 that is placed on the dialysate circuit 141 and feeds liquid in the dialysate circuit 141; a flow path switching unit 155, 166, 176 that is placed on the dialysate circuit 141 and switches the liquid feed mode between the first liquid feed mode and the second liquid feed mode; and a drive control unit 22 that controls the liquid feed pump 173 and the flow path switching unit 155, 166, 176 and performs a liquid feed mode switching operation to switch the liquid feed mode from the second liquid feed mode to the first liquid feed mode, wherein the drive control unit 22 comprises a reception unit 191 that receives a pressure of the blood circuit 121 and a pressure of the dialysate circuit 141, an adjustment amount prediction unit 193 that uses a prediction model M trained by machine learning using pressure of the blood circuit 121, pressure of the dialysate circuit 141 and an error in water removal amount in the liquid feed mode switching operation in the past as training data, and predicts, from the pressure of the blood circuit 121 and the pressure of the dialysate circuit 141, a drive adjustment amount of the liquid feed pump 173 that suppresses the error in water removal amount, and a drive adjustment unit 194 that adjusts drive of the liquid feed pump 173 according to the drive adjustment amount predicted by the adjustment amount prediction unit 193 when the liquid feed mode is switched from the second liquid feed mode to the first liquid feed mode.
   It is thereby possible to reduce the error in water removal amount due to the pressure fluctuation at the time of switching liquid feed mode from the bypass liquid feed mode to the main liquid feed mode.
(8) The blood purification device 101 as defined by (7), wherein the prediction model M is trained by machine learning using the drive adjustment amount of the liquid feed pump 173 in the liquid feed mode switching operation in the past as training data in addition to the pressure of the blood circuit 121, the pressure of the dialysate circuit 141 and the error in water removal amount in the liquid feed mode switching operation in the past. It is thereby possible to more accurately predict the drive adjustment amount of the liquid feed pump that suppresses the water removal error.
(9) The blood purification device 101 as defined by (7) or (8), comprising: a liquid supply pump 163 that is placed on the dialysate supply flow path 152 and feeds the dialysate; a liquid discharge pump 174 that is placed on the dialysate discharge flow path 153 and feeds the dialysate; and an auxiliary pump 173 as the liquid feed pump that is placed on the dialysate discharge flow path 153 on an upstream side of the liquid discharge pump 174 to assist liquid feeding by the liquid discharge pump174, wherein the drive adjustment unit 194 adjusts drive of the auxiliary pump 173 according to the drive adjustment amount predicted by the adjustment amount prediction unit 193 when the liquid feed mode is switched from the second liquid feed mode to the first liquid feed mode.
   It is thereby possible to reduce the water removal error without changing the control of the liquid supply pump and the liquid discharge pump which are cooperatively driven. As a result, a trouble in coordination of the liquid supply pump and the liquid discharge pump caused by changing the control can be prevented.
(10) A method for controlling liquid feed pump 73, comprising: a drive control step of controlling drive of a liquid feed pump 73 of a blood purification device 1 that comprises a blood circuit 21 to circulate blood of a patient C through a blood purifier 10 that purifies the blood, a dialysate circuit 41 which comprises a dialysate supply flow path 52 to supply dialysate toward the blood purifier 10, a dialysate discharge flow path 53 to discharge dialysate from the blood purifier 10, and a bypass flow path 54 connecting the dialysate supply flow path 52 to the dialysate discharge flow path 53, and through which liquid is fed in a liquid feed mode that is either a first liquid feed mode of feeding liquid from the dialysate supply flow path 52 to the dialysate discharge flow path 53 via the blood purifier 10, or a second liquid feed mode of feeding liquid from the dialysate supply flow path 52 to the dialysate discharge flow path 53 via the bypass flow path 54 without passing through the blood purifier 10, the liquid feed pump 73 that is placed on the dialysate circuit 41 and feeds liquid in the dialysate circuit 41, and a flow path switching unit 55, 66, 76 that is placed on the dialysate circuit 41 and switches the liquid feed mode between the first liquid feed mode and the second liquid feed mode, wherein in the drive control step, when a pressure difference occurs between the blood circuit 21 and the dialysate circuit 41 at the time that the flow path switching unit 55, 66, 76 switches the liquid feed mode from the second liquid feed mode to the first liquid feed mode, a command value of the liquid feed pump 73 is adjusted so that the pressure difference is reduced.
   It is thereby possible to reduce the water removal error due to the pressure fluctuation at the time of switching the liquid feed mode from the bypass liquid feed mode to the main liquid feed mode.
(11) A method for controlling liquid feed pump, comprising: a liquid feed mode switching step of performing a liquid feed mode switching operation to switch a liquid feed mode from a second liquid feed mode to a first liquid feed mode in a blood purification device 101 that comprises a blood circuit 121 to circulate blood of a patient C through a blood purifier 110 that purifies the blood, a dialysate circuit 141 which comprises a dialysate supply flow path 152 to supply dialysate toward the blood purifier 110, a dialysate discharge flow path 153 to discharge dialysate from the blood purifier 110, and a bypass flow path 154 connecting the dialysate supply flow path 152 to the dialysate discharge flow path 153, and through which liquid is fed in a liquid feed mode that is either the first liquid feed mode of feeding liquid from the dialysate supply flow path 152 to the dialysate discharge flow path 153 via the blood purifier 110, or the second liquid feed mode of feeding liquid from the dialysate supply flow path 152 to the dialysate discharge flow path 153 via the bypass flow path 154 without passing through the blood purifier 110, a liquid feed pump 173 that is placed on the dialysate circuit 141 and feeds liquid in the dialysate circuit 141, and a flow path switching unit 155, 166, 176 that is placed on the dialysate circuit 141 and switches the liquid feed mode between the first liquid feed mode and the second liquid feed mode, wherein the liquid feed mode switching step comprises performing a reception step S101 of receiving a pressure of the blood circuit 121 and a pressure of the dialysate circuit 141, an adjustment amount prediction step S103 of using a prediction model M trained by machine learning using pressure of the blood circuit 121, pressure of the dialysate circuit 141 and an error in water removal amount in the liquid feed mode switching operation in the past as training data and predicting, from the pressure of the blood circuit 121 and the pressure of the dialysate circuit 141, a drive adjustment amount of the liquid feed pump 173 that suppresses an error in water removal amount, and a drive adjustment step S104 of adjusting a command value of the liquid feed pump according to the drive adjustment amount predicted in the adjustment amount prediction step S103 when the liquid feed mode is switched from the second liquid feed mode to the first liquid feed mode. It is thereby possible to reduce the water removal error due to the pressure fluctuation at the time of switching the liquid feed mode from the bypass liquid feed mode to the main liquid feed mode.

### REFERENCE SIGNS LIST

1: blood purification device, 10: dialyzer, 21: blood circuit,22: main control unit, 41: dialysate circuit, 52: dialysate supply flow path, 53: dialysate discharge flow path, 54: bypass flow path, 55: third electromagnetic valve, 63: liquid supply pump, 66: first electromagnetic valve, 71: second electromagnetic valve, 73: pressure pump, 74: liquid discharge pump, 101: blood purification device, 110: dialyzer, 121: blood circuit, 123: main control unit, 141: dialysate circuit, 152: dialysate supply flow path, 153: dialysate discharge flow path, 154: bypass flow path, 155: third electromagnetic valve, 163: liquid supply pump, 166: first electromagnetic valve, 171: second electromagnetic valve, 173: pressure pump, 174: liquid discharge pump, 191: reception unit, 193: adjustment amount prediction unit, 194: drive adjustment unit, C: patient, M: prediction model

## Claims

1. A blood purification device, comprising:
a blood circuit to circulate blood of a patient through a blood purifier that purifies the blood;
a dialysate circuit which comprises a dialysate supply flow path to supply dialysate toward the blood purifier, a dialysate discharge flow path to discharge dialysate from the blood purifier, and a bypass flow path connecting the dialysate supply flow path to the dialysate discharge flow path, and through which liquid is fed in a liquid feed mode that is either a first liquid feed mode of feeding liquid from the dialysate supply flow path to the dialysate discharge flow path via the blood purifier, or a second liquid feed mode of feeding liquid from the dialysate supply flow path to the dialysate discharge flow path via the bypass flow path without passing through the blood purifier;
a liquid feed pump that is placed on the dialysate circuit and feeds liquid in the dialysate circuit;
a flow path switching unit that is placed on the dialysate circuit and switches the liquid feed mode between the first liquid feed mode and the second liquid feed mode; and
a drive control unit that controls the liquid feed pump and the flow path switching unit,
wherein when a pressure difference occurs between the blood circuit and the dialysate circuit at the time that the flow path switching unit switches the liquid feed mode from the second liquid feed mode to the first liquid feed mode, the drive control unit adjusts drive of the liquid feed pump so that the pressure difference is reduced.

2. The blood purification device according to claim 1, comprising:
a liquid supply pump that is placed on the dialysate supply flow path and feeds the dialysate;
a liquid discharge pump that is placed on the dialysate discharge flow path and feeds the dialysate; and
an auxiliary pump as the liquid feed pump that is placed on the dialysate discharge flow path on an upstream side of the liquid discharge pump to assist liquid feeding by the liquid discharge pump,
wherein when the pressure difference occurs between the blood circuit and the dialysate circuit at the time that the flow path switching unit switches the liquid feed mode from the second liquid feed mode to the first liquid feed mode, the drive control unit adjusts drive of the auxiliary pump so that the pressure difference is reduced.

3. The blood purification device according to claim 1 or 2, wherein the drive control unit acquires a trans-membrane pressure difference in the blood purifier as the pressure difference, and adjusts drive of the liquid feed pump based on the trans-membrane pressure difference at the time that the flow path switching unit switches the liquid feed mode from the second liquid feed mode to the first liquid feed mode.

4. The blood purification device according to claim 1 or 2, wherein when a pressure of the dialysate circuit becomes more than a predetermined threshold value at the time that the flow path switching unit switches the liquid feed mode from the second liquid feed mode to the first liquid feed mode, the drive control unit adjusts drive of the liquid feed pump so that the pressure difference is reduced.

5. The blood purification device according to claim 1 or 2, wherein before the flow path switching unit switches the liquid feed mode from the second liquid feed mode to the first liquid feed mode, the drive control unit adjusts drive of the liquid feed pump so that the pressure difference is reduced.

6. A blood purification device, comprising:
a blood circuit to circulate blood of a patient through a blood purifier that purifies the blood;
a dialysate circuit which comprises a dialysate supply flow path to supply dialysate toward the blood purifier, a dialysate discharge flow path to discharge dialysate from the blood purifier, and a bypass flow path connecting the dialysate supply flow path to the dialysate discharge flow path, and through which liquid is fed in a liquid feed mode that is either a first liquid feed mode of feeding liquid from the dialysate supply flow path to the dialysate discharge flow path via the blood purifier, or a second liquid feed mode of feeding liquid from the dialysate supply flow path to the dialysate discharge flow path via the bypass flow path without passing through the blood purifier;
a liquid feed pump that is placed on the dialysate circuit and feeds liquid in the dialysate circuit;
a flow path switching unit that is placed on the dialysate circuit and switches the liquid feed mode between the first liquid feed mode and the second liquid feed mode; and
a drive control unit that controls the liquid feed pump and the flow path switching unit and performs a liquid feed mode switching operation to switch liquid feed mode from the second liquid feed mode to the first liquid feed mode,
wherein the drive control unit comprises: a reception unit that receives a pressure of the blood circuit and a pressure of the dialysate circuit, an adjustment amount prediction unit that uses a prediction model trained by machine learning using pressure of the blood circuit, pressure of the dialysate circuit and an error in water removal amount in the liquid feed mode switching operation in the past as training data, and predicts, from the pressure of the blood circuit and the pressure of the dialysate circuit, a drive adjustment amount of the liquid feed pump that suppresses the error in water removal amount, and a drive adjustment unit that adjusts drive of the liquid feed pump according to the drive adjustment amount predicted by the adjustment amount prediction unit when the liquid feed mode is switched from the second liquid feed mode to the first liquid feed mode.

7. The blood purification device according to claim 6, wherein the prediction model is trained by machine learning using the drive adjustment amount of the liquid feed pump in the liquid feed mode switching operation in the past as training data in addition to the pressure of the blood circuit, the pressure of the dialysate circuit and the error in water removal amount in the liquid feed mode switching operation in the past.

8. The blood purification device according to claim 6 or 7, comprising:
a liquid supply pump that is placed on the dialysate supply flow path and feeds the dialysate;
a liquid discharge pump that is placed on the dialysate discharge flow path and feeds the dialysate; and
an auxiliary pump as the liquid feed pump that is placed on the dialysate discharge flow path on an upstream side of the liquid discharge pump to assist liquid feeding by the liquid discharge pump,
wherein the drive adjustment unit adjusts drive of the auxiliary pump according to the drive adjustment amount predicted by the adjustment amount prediction unit when the liquid feed mode is switched from the second liquid feed mode to the first liquid feed mode.

9. A method for controlling liquid feed pump, comprising:
a drive control step of controlling drive of a liquid feed pump of a blood purification device that comprises a blood circuit to circulate blood of a patient through a blood purifier that purifies the blood, a dialysate circuit which comprises a dialysate supply flow path to supply dialysate toward the blood purifier, a dialysate discharge flow path to discharge dialysate from the blood purifier, and a bypass flow path connecting the dialysate supply flow path to the dialysate discharge flow path, and through which liquid is fed in a liquid feed mode that is either a first liquid feed mode of feeding liquid from the dialysate supply flow path to the dialysate discharge flow path via the blood purifier, or a second liquid feed mode of feeding liquid from the dialysate supply flow path to the dialysate discharge flow path via the bypass flow path without passing through the blood purifier, the liquid feed pump that is placed on the dialysate circuit and feeds liquid in the dialysate circuit, and a flow path switching unit that is placed on the dialysate circuit and switches the liquid feed mode between the first liquid feed mode and the second liquid feed mode,
wherein in the drive control step, when a pressure difference occurs between the blood circuit and the dialysate circuit at the time that the flow path switching unit switches the liquid feed mode from the second liquid feed mode to the first liquid feed mode, a command value of the liquid feed pump is adjusted so that the pressure difference is reduced.

10. A method for controlling liquid feed pump, comprising:
a liquid feed mode switching step of performing a liquid feed mode switching operation to switch a liquid feed mode from a second liquid feed mode to a first liquid feed mode in a blood purification device that comprises a blood circuit to circulate blood of a patient through a blood purifier that purifies the blood, a dialysate circuit which comprises a dialysate supply flow path to supply dialysate toward the blood purifier, a dialysate discharge flow path to discharge dialysate from the blood purifier, and a bypass flow path through connecting the dialysate supply flow path to the dialysate discharge flow path, and through which liquid is fed in a liquid feed mode that is either the first liquid feed mode of feeding liquid from the dialysate supply flow path to the dialysate discharge flow path via the blood purifier, or the second liquid feed mode of feeding liquid from the dialysate supply flow path to the dialysate discharge flow path via the bypass flow path without passing through the blood purifier, a liquid feed pump that is placed on the dialysate circuit and feeds liquid in the dialysate circuit, and a flow path switching unit that is placed on the dialysate circuit and switches the liquid feed mode between the first liquid feed mode and the second liquid feed mode,
wherein the liquid feed mode switching step comprises performing a reception step of receiving a pressure of the blood circuit and a pressure of the dialysate circuit, an adjustment amount prediction step of using a prediction model trained by machine learning using pressure of the blood circuit, pressure of the dialysate circuit and an error in water removal amount in the liquid feed mode switching operation in the past as training data and predicting, from the pressure of the blood circuit and the pressure of the dialysate circuit, a drive adjustment amount of the liquid feed pump that suppresses the error in water removal amount, and a drive adjustment step of adjusting a command value of the liquid feed pump according to the drive adjustment amount predicted in the adjustment amount prediction step when the liquid feed mode is switched from the second liquid feed mode to the first liquid feed mode.
